# FASCICULE DE BREVET EUROPEEN

(11) **EP 3 817 715 B1**
(45) Date de publication et mention de la délivrance du brevet: **17.05.2023**
(21) Numéro de dépôt: 18765944.6
(22) Date de dépôt: 06.07.2018
(51) Int. Cl.: A61K 8/64, A61Q 19/08, A61K 8/9789, A61Q 19/00

(54) **UTILISATION COSMETIQUE DE HRGP (HYDROXYPROLIN RICH GLYCOPROTEINS) ISSUES DE CELLULES D'AJUGA REPTANS POUR PREVENIR ET/OU LUTTER CONTRE LES EFFETS DU VIEILLISSEMENT DE LA PEAU**
KOSMETISCHE VERWENDUNG VON HRGP (HYDROXYPROLINREICHEN GLYKOPROTEINEN) AUS AJUGA-REPTANS-ZELLEN ZUR VORBEUGUNG UND/ODER BEKÄMPFUNG DER AUSWIRKUNGEN DER HAUTALTERUNG
COSMETIC USE OF HRGP (HYDROXYPROLINE-RICH GLYCOPROTEINS) FROM AJUGA REPTANS CELLS TO PREVENT AND/OR COMBAT THE EFFECTS OF SKIN AGEING

(43) Date de publication de la demande: 12.05.2021
(73) Titulaire: Laboratoires de Biologie Végétale Yves Rocher, 56200 Gacilly (FR)
(72) Inventeur: LAPERDRIX, Céline, 78280 SAINT REMY LES CHEVREUSES (FR); PORTET, Bénédicte, 84450 Saint Saturnin lès Avignon (FR); LUBRANO, Christian, 92310 SEVRES (FR)
(74) Mandataire: Novagraaf Technologies
(86) Numéro de dépôt international: PCT/FR2018/051693
(87) Numéro de publication internationale: WO 2020/008117

(56) Documents cités:
- EP-A1- 0 800 585
- EP-A2- 1 736 166
- EP-B1- 0 800 585
- EP-B1- 1 736 166
- WO-A1-2011/124848
- US-A- 5 882 664
- US-A1- 2011 020 476
- Hijazi May ET AL: "An update on post-translational modifications of hydroxyproline-rich glycoproteins: toward a model highlighting their contribution to plant cell wall architecture", Frontiers in Plant Science, vol. 5, 14 August 2014 (2014-08-14), pages 1-10, XP055844522, DOI: 10.3389/fpls.2014.00395

## Description

### Domaine technique

La présente invention se rapporte à l'utilisation cosmétique de glycoprotéines riches en hydroxyproline (HRGP, « hydroxyprolin rich glycoproteins »), issues de cellules *d'Ajuga reptans,* ainsi qu'à l'utilisation d'une composition cosmétique ou dermatologique comprenant des glycoprotéines riches en hydroxyproline, issues de cellules *d'Ajuga reptans,* et à-un procédé impliquant cette composition.

La présente invention trouve des applications dans le domaine de la cosmétique et de la dermatologie, plus particulièrement de la cosmétique cutanée.

Dans la description ci-dessous, les références entre crochets ([]) renvoient à la liste des références présentée à la fin du texte.

### Etat de la technique

La peau est un organe vital à part entière qui se compose de trois tissus distincts, assumant chacun différents rôles grâce à différents types cellulaires et différentes structures.

Le tissu le plus en surface, et donc le plus exposé, est l'épiderme. Cet épithélium pluristratifié (Malpighien) et kératinisé, se compose de différentes cellules associées à de nombreuses fonctions de barrière et de protection. Les cellules majoritaires sont les kératinocytes qui se divisent dans la couche basale et entament leur différenciation jusqu'à la couche cornée (couche la plus externe), puis sont éliminés par desquamation en 21 à 28 jours en moyenne. Le rôle majeur de l'épiderme est d'apporter à la peau, et donc au corps humain, une première ligne de protection contre les agressions extérieures, comme les agressions physiques, chimiques, hydriques et bactériologiques. Cette protection est notamment assurée par les couches les plus différenciées et la couche cornée connue pour ses propriétés hydrophobes, son aspect compacte et étanche. Une bonne différenciation et donc une bonne desquamation sont essentielles pour une surface lisse, homogène et régulière. L'ensemble de ces mécanismes dépend directement des capacités prolifératives des kératinocytes. Or, les stress extérieurs et l'âge ralentissent ce processus qui peut alors devenir plus long et plus irrégulier. La desquamation devenant irrégulière et/ou anarchique, des défauts de lissage et d'homogénéité de surface de la peau s'installent.

En position intermédiaire, le derme est un tissu conjonctif investi majoritairement de fibroblastes et de protéines matricielles donnant à la peau ses qualités de compressibilité et d'élasticité connues. Les modifications de sa texture et de sa composition sont largement responsables des altérations de la peau qui surviennent au cours du vieillissement. Le relief de la couche cornée est, par ailleurs, directement conditionné par la qualité et la densité du derme qui la sous-tend. Plus que l'épiderme, dont le renouvellement est rapide et constant, le derme subit des troubles importants liés à l'âge, par le vieillissement de ses cellules et de sa matière. En vieillissant, les fibroblastes sont de moins en moins réactifs et de moins en moins prolifératifs. Avec l'âge, les synthèses des macromolécules de la matrice ne sont plus assurées par les fibroblastes. De plus, ces macro-molécules constitutives du derme, telles que les fibres de collagène, les fibres élastiques, les protéoglycanes et les glycosaminoglycanes (acide hyaluronique notamment), ont tendance à dégénérer et à se fragmenter. Leurs réseaux se désorganisent et se réorientent parallèlement à la jonction dermo-épidermique, notamment sous l'action d'enzymes de dégradations des protéines matricielles, les MMP (Matrix Metallo Proteases), aussi appelées collagénases ou élastases. Ces phénomènes d'altération du tissu dermique provoquent en surface un défaut d'organisation de l'épiderme et, d'une façon plus visible et plus directe, une perte de fermeté pouvant aller jusqu'à la ptose cutanée et/ou la formation de rides.

Au sein de la trame conjonctive, s'intercalent aussi d'autres cellules et structures, tel un important réseau circulatoire et nutritif, constitué des vaisseaux sanguins et des capillaires lymphatiques, ainsi que les annexes épidermiques : cheveux, poils, ongles, glandes pilosébacées et glandes sudoripares, qui prennent naissance dans le derme profond.

L'hypoderme, situé en profondeur et constitué en majeure partie de lobules graisseux (adipocytes), assure une fonction de support primaire, de protection mécanique et thermique et joue aussi un rôle de stockage des réserves énergétiques rapidement mobilisables pour tous les besoins biologiques, comme par exemple le renouvellement cellulaire, la défense de l'organisme ou la contraction musculaire.

Les attentes en terme d'anti-âge s'orientent de plus en plus vers des produits naturels, efficaces et éco-conçus. Des actifs comme la vitamine C sont généralement présents dans les produits anti-âge, mais ce composé est très instable et nécessite donc l'ajout de conservateurs ou l'utilisation de dérivés non naturels, peu appréciés des consommateurs.

Il reste donc un réel besoin de trouver de nouvelles compositions et/ou composés permettant d'assurer de façon effective et complète un effet anti-âge au niveau des cellules de la peau, de ses annexes et des muqueuses. En particulier, il existe un réel besoin de trouver des composés naturels qui permettent de prévenir, c'est-à-dire inhiber ou, à tout le moins, retarder ou traiter les effets du vieillissement des structures et des cellules de la peau, de ses annexes et des muqueuses, à la fois en stimulant les cellules de la peau et la synthèse d'éléments de la matrice extracellulaire.

### Description de l'invention

Les inventeurs sont les tout premiers à utiliser des glycoprotéines riches en hydroxyproline (HRGP), issues de cellules *d'Ajuga reptans,* permettant de répondre efficacement aux besoins en termes de diminution du relâchement et/ou de la perte d'élasticité de la peau et/ou de ses annexes et/ou des muqueuses.

Les inventeurs ont en effet découvert que les HRGP issues de cellules *d'Ajuga reptans* possèdent de manière inattendue un effet anti-âge en termes de diminution du relâchement et de la perte d'élasticité de la peau et/ou de ses annexes et/ou des muqueuses.

Les inventeurs ont mis en évidence un effet de ces HRGP sur la synthèse d'élastine et d'acide hyaluronique, sur le renouvellement et le métabolisme des fibroblastes dermiques.

Ainsi, les inventeurs décrivent ces HRGP en tant qu'agent cosmétique anti-âge pour améliorer l'aspect de la peau, notamment par la régénération de la peau et/ou l'amélioration de la fermeté, et/ou de l'élasticité et/ou l'amélioration des propriétés biomécaniques de la peau et de lutter contre les effets visibles de l'âge notamment le relâchement et/ou la perte de fermeté et/ou la perte d'élasticité et/ou la formation des rides. Ces HRGP sont décrits pour permettre également d'apporter un effet cosmétique sur la surface de la peau, notamment de lissage, et/ou de douceur. L'utilisation des HRGP permet avantageusement à la peau de présenter un aspect plus jeune.

*Ajuga reptans,* également appelée bugle rampante, est une plante vivace de la famille des lamiacées originaire de l'Europe, du Caucase et de l'Iran. Elle est commune en France jusque 2 000 mètres d'altitude mais plus rare en région méditerranéenne. C'est une plante rhizomateuse persistante qui produit des stolons permettant une tenue au sol plus forte et favorisant sa propagation. D'une taille de 10 à 40 cm de haut, son étalement peut aller jusqu'à 1 m de diamètre. *Ajuga reptans* est traditionnellement utilisée pour l'ornementation ou pour ces propriétés anti-inflammatoires, notamment pour soulager les douleurs rhumatismales bénignes.

Les glycoprotéines riches en hydroxyproline (HRGP) sont les composants principaux de la paroi pecto-cellulosique des cellules végétales. La paroi des cellules végétales comporte une fraction polysaccharidique, une fraction polypeptidique et des ions minéraux, en particulier du calcium. La fraction polypeptidique comprend des protéines de structure et des protéines enzymatiques. Dans le groupe des HRGP, on distingue les lectines riches en hydroxyproline et les extensines (Quel rôle pour les extensines dans la structure des parois des cellules végétales, Regard sur la biochimie, Cathy Hirsinger, Elisabeth Jamet, 1997 **([1])).** Parmi les protéines de structure, les extensines sont donc des protéines majoritaires et peuvent représenter jusqu'à 15% de la matière sèche de la paroi. L'extensine est une glycoprotéine d'environ 86 kDa, constitué de 35% d'acides aminés et de 65% d'oses. La séquence d'acides aminés des extensines est très répétitive et contient un motif pentapeptidique caractéristique (SER-PRO-PRO-PRO-PRO) dans lequel les résidus proline sont d'abord hydroxylés (riche en hydroxyproline : 33 à 42% du total des acides aminés) puis glycosylés. Elle est pourvue de chaines latérales osidiques (arabinane, galactane) (Physiologie végétale, DUNOD **([2])).** L'extensine contient aussi de la serine, susceptible de se lier à un hexose, le galactose. D'autres acides aminés sont également présents : Lysine, Valine, tyrosine, Thréonine.

Des effets biologiques concernant l'inflammation et la cicatrisation, dus à la présence de polyphénols dans les extraits de Syringa vulgaris et d'Ajuga reptans, précisément de verbascoside et de teupolioside, ont été décrits (Korkina et al. : « Molecular mechanisms underlying wound healing and anti-inflammatory properties of naturally occurring biotechnologically produced phenylpropanoid glycosides », Cell Mol Biol. 2007 May 30;53(5):84-91 **([3])).** Ces polyphénols sont contenus dans le cytoplasme des cellules et ne font en aucun cas partie des molécules de la paroi cellulaire.

Des compositions cosmétiques et pharmaceutiques contenant des extensines ont également été décrites, pour leurs propriétés humectantes et hydratantes (EP0533408 **([4])).**

Ainsi, un premier objet de l'invention se rapporte à l'utilisation cosmétique de HRGP issues de cellules *d'Ajuga reptans,* pour au moins une action choisie parmi une diminution du relâchement et une diminution de perte d'élasticité de la peau et/ou de ses annexes et/ou des muqueuses.

L'action de diminution du relâchement/ perte d'élasticité est notamment une prévention et/ou un retard et/ou une limitation du relâchement, et/ou une action de raffermissement et/ou une amélioration des qualités bio-mécaniques de la peau, notamment favorisant un raffermissement des structures de la peau de la peau, de ses annexes ou des muqueuses.

On entend par « diminution du relâchement/ de la perte d'élasticité » au sens de la présente invention, une action de stimulation de la synthèse d'élastine et d'acide hyaluronique de la peau, de ses annexes et/ou des muqueuses. Avantageusement, cette action peut permettre d'améliorer les qualités biomécaniques du derme, notamment la fermeté, le volume et la résistance.

L'utilisation cosmétique peut avantageusement permettre d'améliorer les qualités de surface, donc esthétiques, de l'épiderme.

On entend par « annexes », au sens de la présente invention, les annexes épidermiques qui prennent naissance dans le derme profond, comprenant au moins un élément choisi parmi les poils, notamment les cheveux et les ongles.

On entend par « muqueuse », au sens de la présente invention, notamment les muqueuses externes comme les lèvres.

On entend par « HRGP issues de cellules *d'Ajuga reptans »,* un extrait comprenant des HRGP issu de cellules *d'Ajuga reptans,* ou des HRGP isolées d'un extrait de parois de cellules *d'Ajuga reptans.* Par exemple, les cellules peuvent être obtenues par un procédé de cultures *in-vitro* de cals de cellules dédifférenciées ou de cals de cellules méristématiques.

Lesdites HRGP peuvent comprendre des glycoprotéiques monomériques, consistant en :
- Des protéines de masse moyenne supérieure à 5 kDa à hauteur de 43 à 76% en poids de la quantité totale des protéines,
- Des protéines de masse moyenne inférieure à 5 kDa à hauteur de 24 à 57% en poids de la quantité totale des protéines.

Avantageusement, ledit extrait est un extrait riche en HRGP, comprenant notamment de 1 à 100% en poids de HRGP. Les cellules *d'Ajuga reptans* peuvent être obtenues à partir de toute technique adaptée connue de l'homme du métier.

Il peut s'agir par exemple d'une culture *in vitro* de cellules *d'Ajuga reptans,* comme décrite par Trevor A.Thorpe (History of plant tissue culture. Molecular biotechnology, 2007, 37, 2, 169-180 **([5]))** et permettant l'obtention de cals soit d'amas de cellules méristématiques indifférenciées, soit d'amas de cellules dédifférenciées. Dans le cas de cellules dédifférenciées, le principe consiste, à partir d'un échantillon d'un organe de la plante, à dédifférencier les cellules constitutives de l'échantillon en faisant croître un cal sur une blessure, tandis que les cellules méristématiques, qui sont des cellules indifférenciées, sont directement prélevées dans les régions méristématiques de la plante. On induit ensuite la multiplication cellulaire de ces cellules dédifférenciées ou méristématiques. Le but est d'accroître la biomasse afin qu'un grand nombre de cellules soient récupérées, entières, ou fragmentées. On peut alors récupérer le contenu cellulaire avec avantageusement une concentration importante en métabolites d'intérêt, ou les macromolécules des parois des cellules, ou conserver l'ensemble. De nombreux avantages existent en ce qui concerne l'utilisation de culture cellulaire *in vitro* pour produire des actifs cosmétiques, en particulier l'impact très faible sur l'environnement grâce à un usage faible voire nul de surfaces agricoles.

Alternativement, les cellules *d'Ajuga reptans* peuvent appartenir à une lignée cellulaire préétablie et pouvant être conservées par un organisme habilité.

A partir de la biomasse obtenue et récoltée de cellules dédifférenciées ou méristématiques *d'Ajuga reptans* entières, toute technique de purification des HRGP adaptée connue de l'homme du métier peut être mise en oeuvre. Il peut s'agir par exemple d'un protocole d'obtention mettant en oeuvre les étapes suivantes :
- casser les cellules végétales par exemple par une lyse mécanique ou chimique, ou par digestion enzymatique, préférentiellement par une lyse acide, à l'aide d'un acide minéral, par exemple H₃PO₄ et HCl, ou d'un acide organique, par exemple l'acide acétique, l'acide lactique ou l'acide citrique, ajouté à la biomasse cellulaire ; lorsqu'une lyse acide est utilisée pour casser les cellules, de préférence une période d'incubation d'au moins 30 min est mise en oeuvre, de préférence d'au moins 2h, de préférence encore d'au moins 6h et de préférence encore plus d'au moins 18h, et à une température inférieure à 25°C, de préférence inférieure à environ 5°C ;
- récupérer les parois cellulaires végétales (PCV) en éliminant les molécules intracellulaires solubles par lavage de l'homogénat cellulaire avec une solution aqueuse, par exemple par diafiltration sur un filtre de 0,2 µm, ou par centrifugation ;
- extraire les HRGP des PCV en ajoutant un sel de manière à former une suspension d'extrait salin enrichi en HRGP ; de préférence par ajout de cations inorganiques ;
- récupérer la suspension d'extrait salin enrichi en HRGP en éliminant les débris de PCV, par toute méthode connue, par exemple par filtration ou par centrifugation.

La suspension est collectée pour un traitement ultérieur. Elle peut par exemple ensuite être simultanément dessalée et concentrée à la concentration souhaitée d'HRGP, par exemple par une ultrafiltration sur une membrane de 10 kDa. Le tampon final de l'extrait HRGP peut être tout tampon adapté connu, par exemple le tampon citrate, 0,05 M, à pH 4.

De préférence la biomasse est récoltée après la phase de prolifération cellulaire exponentielle, pendant la phase de croissance stationnaire, plus précisément de préférence après environ 12-16 jours de culture, de préférence encore à 14 jours de culture de la biomasse, correspondant à une croissance de la biomasse après la phase de prolifération exponentielle de 50% à 90%, de préférence 75% à 80%.

Après l'ultrafiltration avec la membrane de 10 kDa, la solution de retentât, qui constitue l'extrait d'HRGP, peut être lyophilisée.

Avantageusement, l'extrait d'HRGP comprend principalement des glycoprotéines monomériques avec plus de 76% de la masse moyenne protéique mesurée supérieure à 5 kDa et 24% inférieure à la masse moyenne des peptides de 5 kDa. Une analyse de la séquence d'acides aminés peut être effectuée après une étape supplémentaire de précipitation des protéines avec de l'acide trichloracétique pour encore augmenter la pureté de HRGP. La masse moyenne des protéines peut être mesurée par toute technique connue de l'homme du métier, comme par exemple par spectrométrie de masse.

L'extrait de HRGP peut subir ensuite une hydrolyse pour améliorer sa fonctionnalité et sa biodisponibilité cutanée.

Les peptides d'HRGP hydrolysés peuvent être préparés en utilisant des protéases après déglycosylation. L'élimination des branches glycosidiques de la protéine HRGP monomérique peut être obtenue par traitement avec une carbohydrase. La déglycosylation des HRGP permet avantageusement l'accès des protéases, telles que la trypsine, à l'ossature de la protéine pour obtenir des fragments peptidiques de plus petite taille par hydrolyse enzymatique. Une combinaison de carbohydrase et de protéases peut être utilisée pour réduire la taille des peptides des extraits de HRGP, comme décrit dans le document WO2011/124848 **([6]),** qui enseigne comment utiliser une séquence ou une combinaison de carbohydrases et de protéases pour extraire les peptides HRGP des fibres de soja.

Après l'hydrolyse enzymatique avec les carbohydrases et les protéases, la quantité totale de peptides détectés avec une masse moyenne inférieure à 5 kDa passé de 24% à 57,2% et la fraction de peptides ayant une masse moyenne supérieure à 5 kDa est réduite de 76% à 43%.

L'échantillon peut être analysé par toute technique connue de l'homme du métier, par exemple par HPLC-MS / MS après digestion avec de la trypsine, dans laquelle plusieurs peptides sont identifiés comme des fragments d'HRGP: un peptide présent dans l'échantillon extrait est identique à la protéine 1 de Solanum lycopersicum proche de l'extensine et riche en leucine (accession gi | 460408850) dans laquelle la proline est de 19,8% de la masse totale de la séquence d'acides aminés.

Un autre objet de l'invention se rapporte à un procédé cosmétique non thérapeutique choisi parmi une diminution du relâchement et une diminution de perte d'élasticité de la peau et/ou de ses annexes et/ou des muqueuse, comprenant une application sur la peau d'une composition cosmétique ou dermatologique anti-âge, comprenant des HRGP issues de cellules *d'Ajuga reptans* et un véhicule cosmétiquement et/ou dermatologiquement acceptable, ladite composition comprenant 0,00001 à 2% en poids de HRGP *d'Ajuga reptans* par rapport au poids total de la composition.

Un autre objet de l'invention se rapporte à une utilisation d'une composition cosmétique ou dermatologique non thérapeutique anti-âge comprenant des HRGP issues de cellules *d'Ajuga reptans* et un véhicule cosmétiquement et/ou dermatologiquement acceptable, ladite composition comprenant 0,00001 à 2% en poids de HRGP *d'Ajuga reptans* par rapport au poids total de la composition, dans un produit dermatologique destiné à au moins une action choisie parmi une diminution du relâchement et une diminution de perte d'élasticité de la peau et/ou de ses annexes et/ou des muqueuses.

Toutes les caractéristiques des HRGP issues de cellules d'*Ajuga reptans* mentionnées ci-avant pour l'utilisation de l'invention, ainsi que toutes les caractéristiques de l'utilisation des HRGP décrites ci-avant, s'appliquent *mutatis mutandis* à la composition décrite.

On entend par « composition cosmétique », toute composition à visée cosmétique, c'est à dire esthétique, une composition pouvant être mise en contact avec les parties superficielles du corps humain, par exemple l'épiderme, les systèmes pileux et capillaires. Avantageusement, une composition cosmétique permet, exclusivement ou principalement de les protéger, parfumer, maintenir en bon état, modifier leur aspect ou en corriger les défauts superficiels.

Dans la présente, on entend par « composition dermatologique » toute composition à visée dermatologique, c'est à dire une composition pouvant être mise en contact avec les parties superficielles du corps humain, pour un traitement de la peau, des muqueuses, et des phanères, comme les ongles, les cheveux, ou les poils.

Par « véhicule cosmétiquement ou dermatologiquement acceptable », on entend un véhicule adapté pour une utilisation en contact avec des cellules humaines et animales cutanées, en particulier les cellules de l'épiderme, sans toxicité, irritation, réponse allergique indue et similaire, et proportionné à un rapport avantage/risque raisonnable.

Le véhicule cosmétiquement acceptable peut être choisi parmi l'eau, l'allantoïne, la glycérine, le méthylpropanediol ; cette liste n'étant pas limitative.

La composition peut être obtenue par tout procédé approprié connu de l'homme du métier pour la fabrication d'une composition cosmétique ou dermatologique. Il peut s'agir, par exemple d'un simple mélange des HRGP *d'Ajuga reptans* avec les autres composants présents dans la composition cosmétique ou dermatologique. Il peut s'agir alternativement, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'inversion de Phase (TIP), ce procédé étant classiquement utilisé par l'homme de l'art pour obtenir des émulsions huile dans eau dont les gouttelettes dispersées sont particulièrement fines, par exemple avec un diamètre de 0,1 à 1 µm.

La composition cosmétique ou dermatologique peut comprendre avantageusement au moins 0,00001% en poids d'HRGP issues de cellules méristématiques *d'Ajuga reptans* par rapport au poids total de la composition, notamment de 0,00001 à 2% en poids d'HRGP issues de cellules méristématiques *d'Ajuga reptans* par rapport au poids total de la composition. Il peut s'agir par exemple de 0,0001 à 1%, ou de 0,001 à 1%, ou de 0,01 à 1%, ou de 0,1 à 1%, ou de 0,0001 à 2%, ou de 0,001 à 2%, ou de 0,01 à 2%, ou de 0,1 à 2% en poids d'HRGP issues de cellules méristématiques *d'Ajuga reptans* par rapport au poids total de la composition.

La composition cosmétique ou dermatologique peut se trouver sous toute forme appropriée pour une application cosmétique ou dermatologique. Avantageusement, la composition est une composition à usage topique.

Il peut s'agir par exemple d'une composition sous une forme choisie dans le groupe comprenant une émulsion huile dans eau ou eau dans huile ou un mélange de ces émulsions.

La composition cosmétique ou dermatologique peut par exemple se trouver sous une forme choisie dans le groupe comprenant un gel aqueux ou hydroalcoolique, une crème aqueuse ou hydroalcoolique et une lotion aqueuse ou hydroalcoolique. Ces formulations utilisables sont connues dans l'état de la technique par les formulateurs. Dans ces exemples de composition, il suffit d'ajouter les HRGP issues de cellules *d'Ajuga reptans* pour obtenir une composition conforme.

La composition peut être sous une forme choisie parmi un onguent, une crème, une huile, un lait, une pommade, une poudre, un tampon imbibé, une solution, un gel, un sérum, un baume, un beurre, une lotion, une suspension, un savon ou une émulsion.

L'extrait peut être utilisé dans une composition cosmétique ou dermatologique seul ou en combinaison avec d'autres ingrédients biologiquement actifs et/ou de substances permettant notamment d'accompagner l'extrait, de constituer une formulation particulière, de conserver l'extrait actif dans le temps, cette liste n'étant pas limitative. Il peut s'agir en d'autres termes de tout produit de base pouvant se trouver dans les compositions cosmétiques ou dermatologiques classiques.

Un dispositif pouvant se présenter sous une forme choisie parmi un pot, un flacon-pompe, une lingette, un masque, un dispositif transdermique, un patch, un spray, ledit dispositif comprenant un extrait ou une composition tels que définis ci-avant, est décrit.

Dans le cadre du procédé cosmétique selon l'invention, ou de l'utilisation selon l'invention, l'utilisation s'entend d'une utilisation non-thérapeutique, par exemple pour le traitement des peaux normales, c'est-à-dire des peaux saines ne présentant pas un état pathologique, à l'exclusion de toute utilisation thérapeutique.

Ainsi, toute utilisation cosmétique et tout procédé cosmétique selon l'invention sont respectivement des utilisations cosmétiques non-thérapeutiques et procédés cosmétiques non-thérapeutiques.

D'autres avantages pourront encore apparaître à l'homme du métier à la lecture des exemples ci-dessous, donnés à titre illustratif.

### EXEMPLES

### Exemple 1 : Préparation d'extrait d'Ajuga reptans

Un échantillon végétal *d'Ajuga reptans* est soumis à une étape de dédifférenciation des cellules constitutives de l'échantillon par la formation de cals par blessure dudit échantillon, puis à une série d'étapes de culture/stabilisation en milieu liquide d'un cal sélectionné, pour induire la formation d'une biomasse par multiplication cellulaire de ces cellules dédifférenciées (phase de prolifération rapide exponentielle), puis une phase d'augmentation de volume de ces cellules (phase de croissance stationnaire) comme décrit dans Thorpe TA **([5]).**

A partir de la biomasse obtenue de cellules dédifférenciées *d'Ajuga reptans* après 14 jours de culture, les HRGP sont extraites selon le protocole suivant :
- lyse acide: un acide minéral, le H₃PO₄, est ajouté à la biomasse cellulaire à un pH=2;
- incubation de l'homogénat cellulaire pendant 16 heures à 4°C;
- lavage de l'homogénat cellulaire avec de l'eau par diafiltration sur un filtre de 0,2 µm, afin d'éliminer les molécules intracellulaires solubles et d'obtenir une fraction enrichie en parois cellulaires végétales;
- extraction au moyen de 200 mM de CaCl₂ et de L-acide ascorbique (1g / L), ajoutés directement à la fraction en parois cellulaires végétales, puis agitation de la suspension pendant 10 minutes à 4 °C;
- récupération des HRGP : La solution enrichie en HRGP est séparée de la fraction épuisée de parois cellulaires végétales par filtration, et collectée pour un traitement ultérieur. La solution d'HRGP riche en calcium est ensuite être dessalée et concentrée à la concentration souhaitée d'hydroxyproline par une ultrafiltration sur une membrane de 10 kDa. Le tampon final de l'extrait HRGP est le tampon citrate, 0,05 M, à pH 4.
- Après l'ultrafiltration avec la membrane de 10 kDa, la solution de retentât, qui constitue l'extrait d'HRGP, est lyophilisée.

### Exemple 2 : Effet d' HRGP issues de cellules dédifférenciées d'Ajuga reptans obtenues dans l'Exemple 1 sur le renouvellement des fibroblastes dermiques

Les études ont été réalisées sur des fibroblastes dermiques humains normaux, obtenus à partir de plasties abdominales d'un donneur âgé de 63 ans, ensemencés en monocouche dans les conditions de culture adéquates, à savoir dans un milieu DMEM (« Dulbecco's Modified Eagle Medium ») supplémenté à 1% de sérum de veau foetal (SVNN), à une température de 37°C, sous atmosphère à 5% de CO₂ et saturée en humidité. Les cellules ont été traitées avec les HRGP issues de cellules méristématiques *d'Ajuga reptans* (à 5.10-2%, 0,1 %, 0,5%, 1%).

Après 1 à 6 jours de traitement, la viabilité cellulaire a été évaluée par quantification de l'activité métabolique des déshydrogénases mitochondriales, par mesure d'hydrolyse du MTT (« MethylThiazoleTetrazolium »).

La référence positive est le milieu DMEM avec 5% de SVNN.

Les données du tableau I ci-dessous concernent le pourcentage de viabilité des fibroblastes, ayant subi différents traitements.

**Tableau 1 : Pourcentage de viabilité des fibroblastes**

| Durée Traitement (jours) | DMEM 1% SVNN | DMEM | HRGP d'Ajuga reptans | | | |
|---|---|---|---|---|---|---|
| | | 5% SVNN | 0,05% | 0,1% | 0,5% | 1% |
| 1 | 100 | 124 | 105 | 112 | 112 | 104 |
| 3 | 100 | 162 | 116 | 117 | 111 | 115 |
| 6 | 100 | 210 | 126 | 125 | 143 | 136 |

Cet exemple montre que l'adjonction de HRGP issues de cellules méristématiques *d'Ajuga reptans* dans le milieu de culture permet aux fibroblastes de proliférer d'avantage, jusqu'à 43% à 0,5%. Les fibroblastes étant des cellules peu prolifératives ce test valide l'intérêt de l'extrait dans le domaine de l'anti-âge.

### Exemple 3 : Effet de HRGP issues de cellules dédifférenciées d'Ajuga reptans obtenues dans l'Exemple 1 sur la synthèse d'élastine par les fibroblastes.

L'étude de la synthèse de tropoélastine est réalisée sur des cultures monocouches de fibroblastes cultivées dans les conditions de culture adéquates avec du milieu DMEM (milieu de Dulbecco - Invitrogen^{®}), en plaques 96 puits (test réalisé en sextuplicata).

Les cellules sont traitées avec les HRGP issues de cellules dédifférenciées *d'Ajuga reptans* (à 0.2% et 1%) durant 72h. A la fin de l'incubation les tapis cellulaires sont fixés puis marqués avec un anticorps primaire dirigé contre la tropoélastine lui-même révélé par un anticorps secondaire fluorescent (GAR-Alexa 488). L'intensité de la fluorescence induite par le marquage de la tropoélastine est ensuite quantifiée et rapportée au nombre de noyaux (coloration Hoechst 33258). La référence interne positive est le TGF-β (10ng/ml). Les calculs présentés dans le tableau Il suivant sont réalisés à partir du milieu seul (de dilution) rapport à une base 100.

**Tableau II :**

| Eléments testés | Concentrations | Synthèse de tropoélastine |
|---|---|---|
| TGF-β | 10ng/mL | +1275% |
| *HRGP d'Ajuga reptans* | 0,2% | +58% |
| | 1% | +101 % |

L'adjonction de HRGP issues de cellules dédifférenciées *d'Ajuga reptans* à 1% permet de stimuler la synthèse de tropoélastine de 101%, et donc d'améliorer les qualités biomécaniques du derme, notamment l'élasticité.

### Exemple 4 : Effet de HRGP issues de cellules dédifférenciées d'Ajuga reptans obtenues dans l'Exemple 1 sur la synthèse d'acide hyaluronique par les cellules.

L'étude de la synthèse de tropoélastine est réalisée sur des cultures monocouches de fibroblastes cultivées dans les conditions de culture adéquates avec du milieu DMEM (milieu de Dulbecco - Invitrogen^{®}), en plaques 96 puits (test réalisé en sextuplicata).

Les cellules sont traitées avec les HRGP issues de cellules dédifférenciées *d'Ajuga reptans* à 1% durant 72h. A la fin de l'incubation la quantité d'acide hyaluronique est évaluée dans les surnageants à l'aide d'un kit Elisa R&D Systems (Ref. DY3614).

La référence interne positive est le TGF-β (10ng/ml). Les calculs présentés dans le tableau Il suivant sont réalisés à partir du milieu seul (de dilution) rapport à une base 100.

**Tableau II :**

| Eléments testés | Concentrations | Synthèse d'acide hyaluronique |
|---|---|---|
| TGF-β | 10ng/mL | +119% |
| *HRGP d'Ajuga reptans* | 1% | +13% |

L'adjonction d'extrait *d'Ajuga reptans* à 1% permet de stimuler la synthèse d'acide hyaluronique de 13%, et donc d'améliorer les qualités biomécaniques du derme, notamment de fermeté.

### Exemple 5 : Formulation de HRGP issues de cellules dédifférenciées d'Ajuga reptans dans un soin de type sérum

| **Composants** | **Pourcentage (% en poids)** |
|---|---|
| EAU PURIFIEE | qsp 100 |
| EAU DE BLEUET BlO | 1,1 |
| MANGIFERINE | 2 |
| GLYCERINE 100% VEG | 3 |
| GOMME XANTHANE | 0,1 |
| GOMME TARA | 0,2 |
| ALCOOL C16/C18 | 1 |
| ACETATE DE TOCOPHEROL | 0,1 |
| HUILE DE SESAME BIO | 1,1 |
| VITAMINE A | 0,1 |
| VITAMINE F | 0,3 |
| AMIDON DE MAIS | 1 |
| EXTENSINE | 1 |
| ALCOOL AGRICOLE 96% VOL | 6 |

### Exemple 6 : Formulation de HRGP issues de cellules dédifférenciées d'Ajuga reptans dans un soin de jour

| **Composants** | **Pourcentage (% en poids)** |
|---|---|
| EAU PURIFIEE | qsp 100 |
| MANGIFERINE | 2 |
| EAU DE BLEUET BIO SB YR | 1,1 |
| GLYCERINE 100% VEG | 5 |
| ACIDE SORBIQUE | 0,1 |
| METHYLPROPANE DIOL | 4 |
| GOMME XANTHANE standard | 0,15 |
| GOMME TARA | 0,5 |
| CIRES | 2 |
| METHYL GLUCOSE STEARATE | 2 |
| ACETATE DE TOCOPHEROL | 0,1 |
| LECITHINE DE SOJA | 0,2 |
| HUILE DE SESAME BIO DESODORISEE°° | 2 |
| HUILE DE COLZA RAFFINEE | 3 |
| HUILE DE SOJA IP°° | 3 |
| EXTENSINE | 1 |
| HYDROXYDE DE SODIUM | 0,05 |

### Exemple 7 : Formulation de HRGP issues de cellules dédifférenciées d' Ajuga reptans dans un soin de nuit.

| **Composants** | **Pourcentage (% en poids)** |
|---|---|
| EAU PURIFIEE | qsp 100 |
| GOMME XANTHANE | 0,15 |
| BUTYLENE GLYCOL | 3 |
| GOMME TARA | 0,5 |
| GLYCERINE 100% VEG | 6 |
| EAU DE BLEUET BIO | 1,1 |
| ACIDE SORBIQUE | 0,1 |
| ALCOOL C18-C22 | 3 |
| BEURRE DE KARITE | 2 |
| HUILE DE COPRAH | 2 |
| ACETATE DE TOCOPHEROL | 0,1 |
| LECITHINE DE SOJA D 300 | 0,2 |
| HUILE DE SOJA | 4 |
| VITAMINE A | 0,1 |
| VITAMINE F | 0,3 |
| EXTENSINE | 1 |

### REFERENCES BIBLIOGRAPHIQUES

1. Quel rôle pour les extensines dans la structure des parois des cellules végétales, Regard sur la biochimie, Cathy Hirsinger, Elisabeth Jamet, 1997.
2. Physiologie végétale, DUNOD.
3. Korkina et al. : « Molecular mechanisms underlying wound healing and anti-inflammatory properties of naturally occurring biotechnologically produced phenylpropanoid glycosides », Cell Mol Biol. 2007 May 30;53(5):84-91.
4. EP0533408.
5. Thorpe TA : "History of plant tissue culture". Molecular biotechnology, 2007, 37, 2, 169-180.
6. WO2011/124848.

## Revendications

1. Utilisation cosmétique de HRGP issues de cellules *d'Ajuga reptans,* pour au moins une action choisie parmi une diminution du relâchement et une diminution de perte d'élasticité de la peau et/ou de ses annexes et/ou des muqueuses.

2. Utilisation cosmétique selon la revendication 1, dans laquelle lesdites HRGP comprennent des glycoprotéines monomériques, consistant en :
- Des protéines de masse moyenne supérieure à 5 kDa à hauteur de 43 à 76% en poids de la quantité totale de protéines,
- Des protéines de masse moyenne inférieure à 5 kDa à hauteur de 24 à 57% en poids de la quantité totale de protéines.

3. Utilisation selon l'une quelconque des revendications précédentes, dans laquelle lesdites cellules *d'Ajuga reptans* sont des cellules méristématiques ou dédifférenciées.

4. Procédé cosmétique non thérapeutique pour au moins une action choisie parmi une diminution du relâchement et une diminution de perte d'élasticité de la peau et/ou de ses annexes et/ou des muqueuses, comprenant une application sur la peau d'une composition cosmétique ou dermatologique anti-âge comprenant des HRGP issues de cellules *d'Ajuga reptans* et un véhicule cosmétiquement et/ou dermatologiquement acceptable, ladite composition comprenant 0,00001 à 2% en poids de HRGP *d'Ajuga reptans* par rapport au poids total de la composition.

5. Utilisation d'une composition cosmétique ou dermatologique non thérapeutique anti-âge comprenant des HRGP issues de cellules *d'Ajuga reptans* et un véhicule cosmétiquement et/ou dermatologiquement acceptable, ladite composition comprenant 0,00001 à 2% en poids de HRGP *d'Ajuga reptans* par rapport au poids total de la composition, dans un produit dermatologique destiné à au moins une action choisie parmi une diminution du relâchement et une diminution de perte d'élasticité de la peau et/ou de ses annexes et/ou des muqueuses.

## Patentansprüche

1. Kosmetische Verwendung von HRGPs aus *Ajuga reptans-Zellen* für mindestens eine Wirkung, die aus der Verringerung der Hauterschlaffung und der Verringerung des Elastizitätsverlusts der Haut und/oder ihrer Anhängsel und/oder der Schleimhäute ausgewählt wird.

2. Kosmetische Verwendung nach Anspruch 1, wobei die HRGP monomere Glykoproteine umfassen, bestehend aus:
- Proteinen mit einer durchschnittlichen Masse von mehr als 5 kDa im Bereich von 43 bis 76 Gew.-% der Gesamtmenge an Proteinen,
- Proteine mit einer durchschnittlichen Masse von weniger als 5 kDa im Bereich von 24 bis 57 Gew.-% der Gesamtmenge der Proteine.

3. Verwendung nach einem der vorhergehenden Ansprüche, wobei die *Ajuga reptans-Zellen* meristematische oder entdifferenzierte Zellen sind.

4. Nichttherapeutisches kosmetisches Verfahren für mindestens eine Wirkung, die aus der Verringerung der Erschlaffung und der Verringerung des Elastizitätsverlusts der Haut und/oder ihrer Anhangsgebilde und/oder Schleimhäute ausgewählt ist, das das Auftragen einer kosmetischen oder dermatologischen Anti-Ageing-Zusammensetzung auf die Haut umfasst, die HRGPs, die von *Ajuga reptans-Zellen* stammen, und einen kosmetisch und/oder dermatologisch akzeptablen Träger enthält, wobei die Zusammensetzung 0,00001 bis 2 Gew.-% HRGPs von *Ajuga reptans,* bezogen auf das Gesamtgewicht der Zusammensetzung, enthält.

5. Nichttherapeutische Verwendung einer kosmetischen oder dermatologischen Anti-Ageing-Zusammensetzung, die HRGPs, die von *Ajuga reptans-Zellen* stammen, und einen kosmetisch und/oder dermatologisch akzeptablen Träger enthält, wobei die Zusammensetzung 0,00001 bis 2 Gew.-% HRGPs von *Ajuga reptans,* bezogen auf das Gesamtgewicht der Zusammensetzung, enthält, in einem dermatologischen Produkt, das für mindestens eine Wirkung bestimmt ist, die unter der Verringerung der Hauterschlaffung und der Verringerung des Elastizitätsverlusts der Haut und/oder ihrer Anhangsgebilde und/oder Schleimhäute ausgewählt wird.

## Claims

1. Cosmetic use of HRGPs from *Ajuga reptans* cells, for at least one action chosen among a reduction in sagging and a reduction in the loss of elasticity of the skin and/or its appendages and/or the mucous membranes.

2. Cosmetic use according to claim 1, wherein said HRGP comprise monomeric glycoproteins, consisting of :
- Proteins with an average mass of more than 5 kDa in the range of 43 to 76% by weight of the total amount of proteins,
- Proteins with an average mass of less than 5 kDa in the range of 24 to 57% by weight of the total amount of proteins.

3. Use according to any one of the preceding claims, wherein said *Ajuga reptans* cells are meristematic or dedifferentiated cells.

4. Non-therapeutic cosmetic method for at least one action chosen among a reduction in sagging and a reduction in loss of elasticity of the skin and/or its appendages and/or mucous membranes, comprising an application to the skin of an anti-ageing cosmetic or dermatological composition comprising HRGPs derived from *Ajuga reptans* cells and a cosmetically and/or dermatologically acceptable vehicle, the said composition comprising 0.00001 to 2% by weight of *Ajuga reptans* HRGPs relative to the total weight of the composition.

5. Non-therapeutic use of an anti-ageing cosmetic or dermatological composition comprising HRGPs derived from *Ajuga reptans* cells and a cosmetically and/or dermatologically acceptable vehicle, the said composition comprising 0.00001 to 2% by weight of *Ajuga reptans* HRGPs relative to the total weight of the composition, in a dermatological product intended for at least one action chosen among a reduction in the sagging and a reduction in the loss of elasticity of the skin and/or its appendages and/or mucous membranes.
